# EUROPEAN PATENT SPECIFICATION

(11) **EP 3 536 367 B1**
(45) Date of publication and mention of the grant of the patent: **11.08.2021**
(21) Application number: 18160818.3
(22) Date of filing: 08.03.2018
(51) Int. Cl.: A61M 15/00

(54) **MEDICATION INHALER FOR AEROSOL PULMONARY DELIVERY WITH SPECIFIC PATTERN RECOGNIZABLE BY AN ELECTRONIC DEVICE**
ARZNEIMITTELINHALATOR ZUR AEROSOL-LUNGENVERABREICHUNG MIT SPEZIFISCHEM, DURCH EINE ELEKTRONISCHE VORRICHTUNG ERKENNBAREM MUSTER
INHALATEUR DE MÉDICAMENT POUR ADMINISTRATION PULMONAIRE D'AÉROSOL AVEC MOTIF SPÉCIFIQUE RECONNAISSABLE PAR UN DISPOSITIF ÉLECTRONIQUE

(43) Date of publication of application: 11.09.2019
(73) Proprietor: Air Liquide Medical Systems S.r.l., 20148 Milano (IT)
(72) Inventor: POLLINI, Marco, 25073 Bovezzo (IT); RUOCCO, Alessandra, 25030 Orzivecchi (IT); ALBERICI, Luca, 25030 RONCADELLE (IT)
(74) Representative: Air Liquide

(56) References cited:
- WO-A1-2012/038861
- FR-A1- 3 021 544
- US-A- 6 039 042
- US-A1- 2012 318 261
- US-A1- 2013 063 579
- US-A1- 2015 339 953

## Description

The present invention relates to an aerosoltherapy assembly comrpising a medication inhaler useable with an aerosol drug delivery (ADD) device, such as a metered dose inhaler (MDI), for delivering metered or pre-metered aerosolized doses of a medicinal product to the human respiratory system of pediatric patients or the like. The medication inhaler comprises a specific pattern on its outer bottom surface that is readable or recognizable by an electronic device, such as a smartphone or the like.

Generally speaking, aerosol drug delivery (ADD) device uses a medication/drug canister and a pressurized gas to disperse a medication or a drug to be inhaled by a patient, in the form of micro-particles or droplets. One common example of ADD device is the well-known metered dose inhaler (MDI). ADD can be reusable or disposable hand-held single- or multi-dose devices.

An ADD device is usually used in combination with a medication inhaler for delivering an inhalable aerosol containing the micro-particles or -droplets generated by the ADD device, to the lungs of the patient.

A medication inhaler comprises an elongated hollow holding chamber comprising an inlet opening into which the ADD device, e.g. MDI, is plugged, and an oulet opening in fluid communication with a respiratory interface, such as a respiratory mask or mouthpiece. The ADD device delivers into the hollow holding chamber, the medication or drug to be inhaled so that micro-particles or -droplets are created into said hollow holding chamber before being inhaled by the patient in need thereof.

A diaphragm valve is generally arranged adjacent to the respiratory interface and designed to open as the patient inhales so as to allow a passage of medication from the hollow holding chamber to the lungs of the patient. Several breaths are often required for the administration of the required drug dosage.

Assemblies comprising medication inhalers and MDIs are disclosed by document EP-A-2014325 and WO-A-03/097142.

Medication inhalers are very efficient if the users, i.e. the patients, accept and follow their inhalation therapy.

However, it has been noted in practice that it is not or rarely the case for a given population of patients, namely pediatric patients, such as children, toddlers or the like.

Indeed, a lot of pediatric patients are either scared by medication inhalers and/or unable to stay calm while a medication inhaler is applied on their face, and totally refuse to inhale the aerosol contained therein.

This obviously leads to a lack of efficiency of the inhalation therapy, especially when said inhalation therapy has, for being efficient, to be delivered for several days or weeks.

Documents US6,039,042 and US2012/318261 disclose medication inhalers for administrating aerosolized drugs comprising bottoms with outer geometrical forms, such as radial spokes, voids or similar, forming a specific pattern.

It is an objective of the present invention to try to overcome the above problem in improving the acceptance of medication inhalers by pediatric patients or the like.

A solution according to the present invention is an aerosoltherapy assembly comprising a medication inhaler comprising a housing defining a hollow chamber, said housing further comprising a bottom cover and a top cover, said bottom cover comprising an intlet for plugging an aerosol drug delivery (ADD) device thereto, said top cover comprising an outlet in fluid communication with the hollow chamber, said bottom cover further comprising an outer bottom surface, a specific pattern being arranged on said outer bottom surface, said specific pattern being readable or recognizable by an electronic device, characterized in that it further comprises an electronic device comprising an embedded camera and a display screen configured for:
a) recognizing or reading the specific pattern carried by the medication inhaler by means of the embedded camera,
b) running an image modification software, such as an app, in response of a recognition or a reading of step a), and
c) live displaying on the display screen by means of the image modification software, a composite image comprising a live image of the user's face and at least a dynamic content superimposed on and/or around the live image of the user's face.

The medication inhaler for aerosol therapry, i.e. the aerosol inhalation device, of the aerosoltherapy assembly according to the present invention can comprise one or several of the following additional features :
- the specific pattern is readable by an electronic device comprising an embedded camera and a display screen, preferably a colour screen.
- the specific pattern is readable by an electronic device comprising a touch screen.
- the specific pattern readable by a smartphone, an electronic pad or the like.
- the specific pattern comprises geometrical or non-geometrical forms.
- the specific pattern comprises several forms having different colors or contrasts, at least two different colors or contrasts, for being easily recognized or read by the electronic device, for example black and white, or yellow and black...
- the specific pattern comprises geometrical or non-geometrical forms having a precise orientation, so that they are univocally located in the space, thereby allowing the electronic device recognizing the correct orientation of the medication inhaler in the space.
- the housing further comprising a bottom end and a top end.
- the bottom cover is fixed to the bottom end of the housing.
- the top cover is fixed to the top end of the housing.
- the bottom cover is detachably fixed to the bottom end of the housing, i.e. removable.
- the top cover is detachably fixed to the top end of the housing, i.e. removable.
- the bottom cover and/or the top cover are detachably fixed to the housing by a snap-fit connection, a screw connection or any other kind of locking mechanism.
- the housing, the bottom cover and the top cover are made of polymer material, such as plastic or the like.
- a one-way valve is arranged between the hollow chamber and the top cover.
- the one-way valve is a duck-beak valve.
- the one-way valve is sandwiched between the hollow chamber and the top cover.
- the one-way valve is a duck-beak valve made of a flexible material.
- the one-way valve is made of a polymeric material, such as silicone, elastomer, rubber or the like.
- the one-way valve is molded in one-piece.
- the one-way valve comprises a peripheral collar traversed by several holes, preferably at least 3 holes.
- the one-way valve comprises at least one slot for allowing the aerosolized flow passing therethrough.
- the housing comprises several pins. The pins are lodged into the holes of the collar of the one-way valve, when the one-way valve is positioned/arranged on the housing, typically sandwiched between the housing and the top cover, for thereby maintaining the one-way valve in a defined and fixed position on the housing.
- the hollow chamber of the housing has a general tubular shape, such as a tronconical or cylindrical shape.
- the hollow chamber is elongated.
- a respiratory interface, such as a respiratory mask or mouthpiece, is fixed to the top cover, preferably detachably fixed thereto.
- the outlet of the top cover is in fluid communication with a respiratory interface, such as a respiratory mask or mouthpiece.
- the intlet of the bottom cover constitutes a central opening for plugging an ADD device thereto.
- the intlet of the bottom cover is configured and sized for receiving and holding an ADD device.
- the top cap further comprises one or several venting orifice(s) comprising venting valve(s) for allowing gases expired by the user to flow out of the medication inhaler.
- it further comprises a whistle element.
- the whistle element is arranged in the gas flow, between the top cover and the housing.

Preferably, the electronic device of the aerosoltherapy assembly according to the present invention is a smartphone.

Advantageously, step c) of live displaying a composite image on the display screen occurs while the user, i.e. the patient, is breathing gas and/or aerosol into the medication inhaler, i.e. inhaling the aerosoltherapy.

The image modification software is configured for moving and/or acting one or several dynamic contents close to, for instance on or around, the live image of the user's face, such as a baby patient or the like, thereby capturing his/her attention and improving the acceptation of the treatment, without crying or the like.

According to the present invention, the image modification software operates a live modification of the live image of the user's face in adding an (or several) additional dynamic content(s).

In preferred embodiments, the aerosoltherapy assembly further comprises an aerosol drug delivery (ADD) device plugged into the medication inhaler.

The present invention will be explained in more details in the following illustrative description of an embodiment of a medication inhaler for aerosoltherapy of an aerosoltherapy assembly according to the present invention, which is made in references to the accompanying drawings among them :
- Figures 1 and 2 represent side views of an embodiment of a medication inhaler of an aerosoltherapy assembly according to the present invention,
- Figure 3 represent an exploded view of the medication inhaler of Figures 1 and 2,
- Figure 4 represents a lateral view of the medication inhaler of Figures 1 and 2,
- Figure 5 represents an embodiment of the outer bottom surface of the bottom cover of the medication inhaler of Figure 4, and
- Figure 6 shows the recognition of a specific pattern carried by the medication inhaler by an electronic device, such as a smartphone.

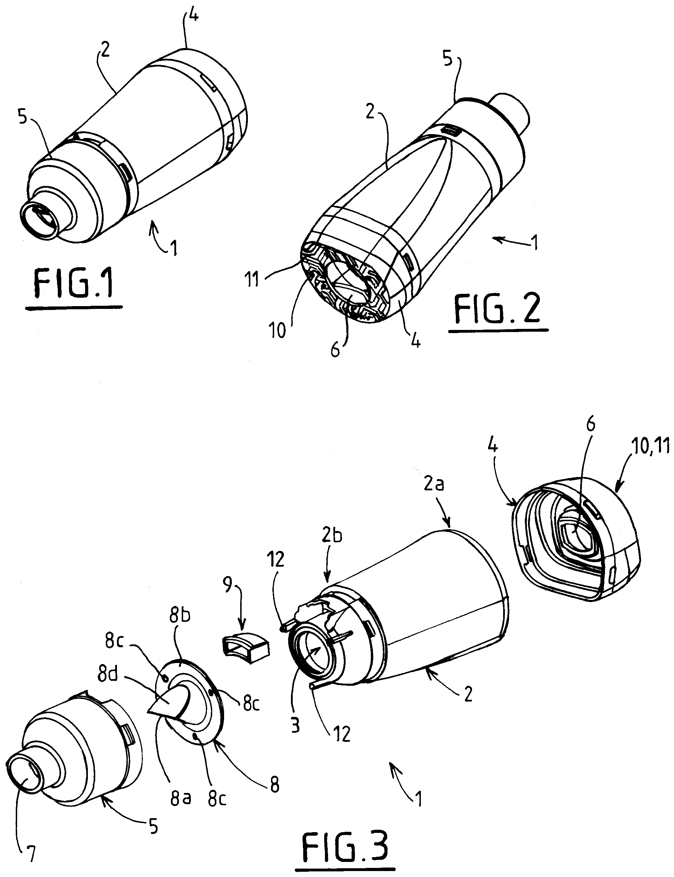

Figures 1 and 2 represent side views of an embodiment of a medication inhaler 1 of an aerosoltherapy assembly according to the present invention, also called a "spacer", that is suitable for aerosol drug pulmonary delivery.

It comprises a housing 2 defining an ellongated hollow chamber 3, and comprising a bottom cover 4 and a top cover 5, that are detachable. It has general tubular shape, such as a tronconical or cylindrical shape as shown in Figures 1-4.

The bottom cover 4 and/or the top cover 5 are detachably fixed to the housing by a snap-fit connection, a screw connection or any other kind of locking mechanism.

The bottom cover 4 comprises an intlet 6 for plugging an aerosol drug delivery (ADD) device thereto, as shown in Figures 2, 5 and 6, whereas the top cover 5 comprises an outlet 7 in fluid communication with the hollow chamber 3 as shown in Figure 1 and 3.

This means that the intlet 6 of the bottom cover 4 constitutes a central opening for plugging an ADD device thereto. In this aim, the intlet 6 of the bottom cover 4 is configured and sized, i.e. structured and/or designed, for receiving and holding an ADD device that is plugged therein.

Preferably, the housing 2, the bottom cover 4 and the top cover 5 are made of polymer material, such as plastic or the like.

Furthermore, as illustrated in Figure 3, a one-way valve 8 is arranged, e.g. sandwiched, between the housing 2 and the top cover 5, preferably a duck-beak valve. Said one-way valve 8 is made of a flexible material, such as silicone, elastomer, rubber or the like. Preferably, the duck-beak valve 8 comprises a peripheral collar 8b traversed by several holes 8c, preferably at least 3 holes, as shown in Figure 3. Further, the valve 8 comprises at least one slot 8a for allowing the aerosolized flow exiting the ellongated hollow chamber 3, to pass therethrough, in the direction of the outlet 7 of the top cover 5 so as to reach the user's airways. Valve 8 is preferably molded in one-piece.

Further, the housing 2 comprises several pins 12, picots or the like. The pins 12 are lodged into the holes 8c of the collar 8b of the valve 8, when the valve 8 is positioned/arranged on the housing 2, typically sandwiched between the housing 2 and the top cover 5, for thereby maintaining the valve 8 in a defined and fixed position on the housing 2, as shown in Figure 3.

The outlet 7 of the top cover 5 is in fluid communication with a respiratory interface (not shown), such as a respiratory mask or mouthpiece, that is detachably fixed to the top cover 5.

Preferably, the top cap 5 can also comprise one or several venting orifice(s) comprising venting valve(s) for allowing gases expired by the user to flow out of the medication inhaler, i.e. to be vented to the atmosphere.

Avantageously, the housing 2 further comprises a whistle element 9 that is arranged, in the gas flow, between the top cover 5 and the housing 2. The whistle 9 is used to have a feedback on the accomplishment of the respiratory acts. When the patient inhales through the inhaler 1 and then exhales gases, the exhaled gases pass through valve 8 and whistle element 9, which produces a whistling sound. This allows the patient and/or other people, such as the medical staff or members of patient's family, to count the number of performed breaths as they correspond to the number of whistles that can be heard over a time period of time, i.e. while the medication is delivered thanks to the inhalation device 1.

As already explained, a lot of pediatric patients, such as babies, toddlers or young infants, are either scared by medication inhalers and/or unable to stay calm while a medication inhaler is applied on their face. As a consequence, they totally refuse to inhale the aerosol which leads to a lack of efficiency of the inhalation therapy, especially when said inhalation therapy has, for being efficient, to be delivered for several days or weeks.

Hence, in the aim of improving the acceptance of medication inhalers by pediatric patients or the like, thereby ensuring a better delivery of the medication of the aerosolized drug, the bottom cover 4 has been modified.

Indeed, according to the present invention, the bottom cover 4 comprises an outer bottom surface 10 carrying a specific pattern 11 that is readable and/or recognizable by an electronic device 20 comprising an embedded camera 21 and a display screen 22, such as a smartphone, a pad/tablet or the like.

The specific pattern 11 preferably comprises geometrical or non-geometrical forms.

The specific pattern comprises fomes 11 that are preferably repeated several times and/or univocally located in space.

Advantageously, the specific pattern 11 comprises colors in strong chromatic contrast, especially with the rest of the background surface, to be easily recognized by the electronic device 20.

The specific pattern 11 can be directly printed or the like on the outer surface 10 of the bottom cover 4 or can be carried by a sticker or similar that is glued or fixed to it.

Alternatively, the specific pattern 11 can also be obtained by a molding procedure based on a combination of different materials and/or colors.

In use, the user first inserts a source of an aerosol medicament or drug, such as an MDI, in the central opening 6 of the bottom cap 4 of the inhaler 1 of the aerosoltherapy assembly of the present invention and releases some aerosol medication into the hollow chamber 3 of the housing 2.

Then, the user starts to inhale the aerosol drug by means of a patient interface, such as a mouthpiece, a mask or similar, plugged into the outlet 7 of the top cap 5. The user's inspiration causes a pressure drop and air will be sucked into the hollow chamber 3 of housing 2, thereby initiating an air flow into the hollow chamber 3 defined by the housing 2 and in the direction of the top cover 5 and of the patient interface, during all the time the patient is inhaling.

The air flow mixes with the drug aerosol and carries the mixture into the hollow chamber 3 of housing 2 and the air/aerosol gaseous mixture, which circulates in the direction of the user's airways, reaches and passes through duck-beak valve 8 and afterwards reaches the patient interface and then the patient's airways.

Then, user ceases to inhale and the expiration phase starts. During the expiration phase, the patient expires CO₂-rich gases that enter into the top cover 5 through the outlet 7. However, the expired gases can not reach the inner chamber of the housing 2 as they are blocked by valve 8 and vented to the atmosphere.

Preferably, the duck-beak valve 8 is a one-way valve. It comprises a valve body 8d having a duck beak shape and comprising a peripheral collar 8b forming a skirt that is traversed by several holes 8c. The duck-beak valve 8 further comprises one (or several) slot 8a allowing the aerosolized composition to traverse it in the direction of the respiratory interface, such as a mouthpiece or a nasal or facial mask. The valve body 8d including the peripheral collar 8b is preferably thin and made of a flexible material, such as silicon, rubber, or the like. Preferably, the flexible material, such as elastomer, rubber or silicon , e.g. by molding.

Preferably, valve 8 works both as an inspiration and expiration valve. When the patient exhales, the orifice 8a closes and the body of the valve 8b between the various pins 8c goes down letting the expired gases passing so that the structure of the inhaler can convey them to the whistle element 9.

When the user is a pediatric patient, such as a baby, a toddler or a young child, it is mandatory to divert his/her attention so that he/she forgets that he/she is inhaling into an inhaler 1, and better accepts of the use of the inhaler 1.

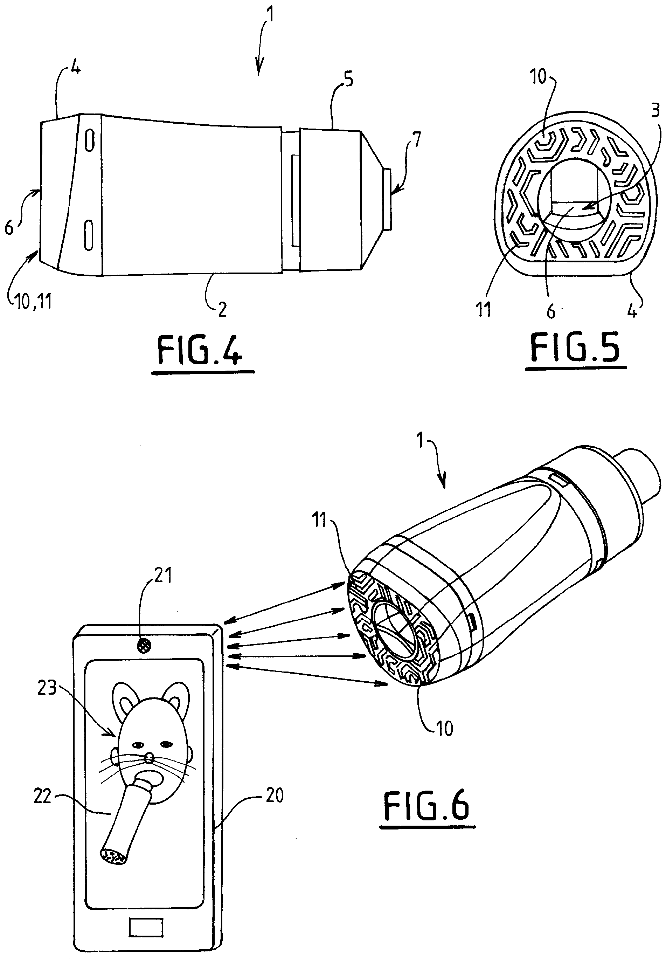

For doing so, an electronic device 20 comprising an embedded camera 21 and a display screen 22, such as a colour touch screen, is used by a second person, for example one of the parents or a medical staff, such as a nurse or a physician. Preferably, the electronic device 20 is a smartphone as shown in Figure 6.

Said electronic device 20, namely its microprocessor or similar, runs a specific software, such as an "app", that uses some ressources of the smartphone, in particular the embedded camera 21 and the display screen 22. By means of the embedded camera 21, the electronic device 20 recognizes and/or read the specific pattern 11, such as curves, waves, dots or any other suitable shapes, and in response, displays a composite image 23 on the display screen 22.

Said composite image 23 is preferably a live picture of the user's face, while he/she is inhaling the aerosol by means of the medication inhaler 1 of the aerosoltherapy assembly of the present invention, that is "modified" by the specific software, in particular an image modification software, of the electronic device 20.

Preferably, the image modification software or "app" does not really transform or change the face of the patient, but rather does add one or several dynamic contents, such as animals, objects, figures, environmental elements... on the image appearing on the screen 22 of the electronic device 20, which includes the user's face.

Said several dynamic contents, also called augmented reality features, are generally put in motion, on or close to the patient's face shown in the screen 22 thereby distracting the patient, while the therapy is delivered by the medication inhaler 1.

The augmented reality features start only when the camera 21 of the electronic devices 20 automatically recognizes the presence of the medication inhaler 1, thanks to the presence of the specific pattern 11 on its bottom outer surface 10.

For instance, the camera can show a live composite picture 23 taken by the video camera 21 of the smartphone 20 comprising a live picture of the baby/child's face, while he/she is inhanling into the housing 2, with additional funny elements, such as animal ears, nose... so that the baby/child appears on the display 22 with a transformed face, e.g. a "funny" face

When the baby/child sees his/her "funny" face, his/her attention is automatically diverted and he/she starts to inhale the aerosol without crying or refusing it.

Aerosoltherapy assembly comprising medication inhalers according to the present invention can greatly improve the acceptace of aerosoltherapies delivered to some populations of patients, especially paediatric ones.

## Claims

1. Aerosoltherapy assembly (1, 20) comprising a medication inhaler (1) comprising a housing (2) defining a hollow chamber (3), said housing (2) further comprising a bottom cover (4) and a top cover (5), said bottom cover (4) comprising an inlet (6) for plugging an aerosol drug delivery (ADD) device thereto, said top cover (5) comprising an outlet (7) in fluid communication with the hollow chamber (3), said bottom cover (4) further comprising an outer bottom surface (10), a specific pattern (11) being arranged on said outer bottom surface (10), said specific pattern (11) being readable or recognizable by an electronic device (20),
**characterized in that** it further comprises an electronic device (20) comprising an embedded camera (21) and a display screen (22) configured for:
a) recognizing or reading the specific pattern carried by the medication inhaler (1) by means of the embedded camera (21),
b) running an image modification software, such as an app, in response of a recognition or a reading of step a), and
c) live displaying on the display screen by means of the image modification software, a composite image comprising a live image of the user's face and at least a dynamic content superimposed on and/or around the live image of the user's face.

2. Aerosoltherapy assembly according to the preceding claim, **characterized in that** the housing (2) of the medication inhaler (1) comprises a bottom end (2a) and a top end (2b), the bottom cover (4) being detachably fixed to the bottom end (2a) and/or the top cover (5) being detachably fixed to the top end (2b).

3. Aerosoltherapy assembly according to any one of the preceeding claims, **characterized in that** the specific pattern (11) is readable by an electronic device (20) comprising an embedded camera (21) and a display screen (22).

4. Aerosoltherapy assembly according to any one of the preceeding claims, **characterized in that** the specific pattern (11) is readable by a smartphone or an electronic pad.

5. Aerosoltherapy assembly according to any one of the preceeding claims, **characterized in that** the specific pattern (11) comprises geometrical or non-geometrical forms.

6. Aerosoltherapy assembly according to any one of the preceeding claims, **characterized in that** the specific pattern (11) comprises several forms having different colors or contrasts for being easily recognized or read by the electronic device (20).

7. Aerosoltherapy assembly according to any one of the preceeding claims, **characterized in that** the specific pattern (11) comprises geometrical or non-geometrical forms having a precise orientation, thereby allowing the electronic device (20) recognizing the correct orientation of the medication inhaler (1) in the space.

8. Aerosoltherapy assembly according to any one of the preceeding claims, **characterized in that** a one-way valve (8) of the medication inhaler (1) is arranged between the housing (2) and the top cover (5).

9. Aerosoltherapy assembly according to claim 8, **characterized in that** the one-way valve (8) of the medication inhaler (1) is a duck-beak valve.

10. Aerosoltherapy assembly according to any one of the preceeding claims, **characterized in that** the medication inhaler (1) further comprises a whistle element (9).

11. Aerosoltherapy assembly according to claim 10, **characterized in that** the whistle element (9) is arranged in the gas flow, between the top cover (5) and the housing (2) of the the medication inhaler (1).

12. Aerosoltherapy assembly according to Claim 1, **characterized in that** the electronic device (20) is a smartphone.

13. Aerosoltherapy assembly according to Claim 12, **characterized in that** the smartphone comprises a microprocessor running a specific software that uses ressources of the smartphone including the embedded camera (21) and the display screen (22) for recognizing and/or reading the specific pattern (11) and in response, displays a composite image (23) on the display screen (22) comprising a live picture of the user's face, while inhaling the aerosol by means of the medication inhaler (1), that is "modified" by the specific software for adding one or several dynamic contents, such as animals, objects, figures or environmental elements, on the image appearing on the display screen (22).

14. Aerosoltherapy assembly according to Claim 1, **characterized in that** it further comprises an aerosol drug delivery (ADD) device plugged into the medication inhaler (1).

## Patentansprüche

1. Anordnung (1, 20) zur Aerosoltherapie, einen Medikationsinhalator (1) umfassend, der ein Gehäuse (2) umfasst, das eine Hohlkammer (3) definiert, wobei das Gehäuse (2) ferner eine untere Abdeckung (4) und eine obere Abdeckung (5) umfasst, wobei die untere Abdeckung (4) einen Einlass (6) zum Anstecken eines Geräts zur Aerosolmedikamentenabgabe (ADD) daran umfasst, wobei die obere Abdeckung (5) einen Auslass (7) in Fluidverbindung mit der Hohlkammer (3) umfasst, wobei die untere Abdeckung (4) ferner eine untere Außenfläche (10) umfasst, wobei auf der unteren Außenfläche (10) ein spezielles Muster (11) angeordnet ist, wobei das spezielle Muster (11) für ein elektronisches Gerät (20) lesbar oder erkennbar ist,
**dadurch gekennzeichnet, dass** sie ferner ein elektronisches Gerät (20) umfasst, das eine eingebettete Kamera (21) und einen Anzeigebildschirm (22) umfasst und für Folgendes konfiguriert ist:
a) Erkennen oder Lesen des speziellen Musters, das der Medikationsinhalator (1) trägt, mittels der eingebetteten Kamera (21),
b) Ausführen einer Bildmodifizierungs-Software, wie beispielsweise einer App, in Reaktion auf ein Erkennen oder Lesen von Schritt a) und
c) Live-Anzeigen eines zusammengesetzten Bildes, das ein Live-Bild des Gesichts eines Benutzers und mindestens einen dynamischen Inhalt umfasst, der auf und/oder rings um das Live-Bild des Gesichts des Benutzers eingeblendet wird, auf dem Anzeigebildschirm mittels der Bildmodifizierungs-Software.

2. Anordnung zur Aerosoltherapie nach dem vorhergehenden Anspruch, **dadurch gekennzeichnet, dass** das Gehäuse (2) des Medikationsinhalators (1) ein unteres Ende (2a) und ein oberes Ende (2b) umfasst, wobei die untere Abdeckung (4) lösbar an dem unteren Ende (2a) befestigt ist und/oder die obere Abdeckung (5) lösbar an dem oberen Ende (2b) befestigt ist.

3. Anordnung zur Aerosoltherapie nach einem der vorhergehenden Ansprüche, **dadurch gekennzeichnet, dass** das spezielle Muster (11) für ein elektronisches Gerät (20) lesbar ist, das eine eingebettete Kamera (21) und einen Anzeigebildschirm (22) umfasst.

4. Anordnung zur Aerosoltherapie nach einem der vorhergehenden Ansprüche, **dadurch gekennzeichnet, dass** das spezielle Muster (11) für ein Smartphone oder ein elektronisches Pad lesbar ist.

5. Anordnung zur Aerosoltherapie nach einem der vorhergehenden Ansprüche, **dadurch gekennzeichnet, dass** das spezielle Muster (11) geometrische oder nichtgeometrische Formen umfasst.

6. Anordnung zur Aerosoltherapie nach einem der vorhergehenden Ansprüche, **dadurch gekennzeichnet, dass** das spezielle Muster (11) mehrere verschiedene Formen umfasst, die unterschiedliche Farben oder Kontraste zum leichten Erkennen oder Lesen durch das elektronische Gerät (20) aufweist.

7. Anordnung zur Aerosoltherapie nach einem der vorhergehenden Ansprüche, **dadurch gekennzeichnet, dass** das spezielle Muster (11) geometrische oder nichtgeometrische Formen umfasst, die eine genaue Ausrichtung aufweisen, wodurch es dem elektronischen Gerät (20) ermöglicht wird, die richtige Ausrichtung des Medikationsinhalators (1) im Raum zu erkennen.

8. Anordnung zur Aerosoltherapie nach einem der vorhergehenden Ansprüche, **dadurch gekennzeichnet, dass** zwischen dem Gehäuse (2) und der oberen Abdeckung (5) ein Rückschlagventil (8) des Medikationsinhalators (1) angeordnet ist.

9. Anordnung zur Aerosoltherapie nach Anspruch 8, **dadurch gekennzeichnet, dass** das Rückschlagventil (8) des Medikationsinhalators (1) ein Entenschnabelventil ist.

10. Anordnung zur Aerosoltherapie nach einem der vorhergehenden Ansprüche, **dadurch gekennzeichnet, dass** der Medikationsinhalator (1) ferner ein Pfeifenelement (9) umfasst.

11. Anordnung zur Aerosoltherapie nach Anspruch 10, **dadurch gekennzeichnet, dass** das Pfeifenelement (9) in dem Gasstrom zwischen der oberen Abdeckung (5) und dem Gehäuse (2) des Medikationsinhalators (1) angeordnet ist.

12. Anordnung zur Aerosoltherapie nach Anspruch 1, **dadurch gekennzeichnet, dass** das elektronische Gerät (20) ein Smartphone ist.

13. Anordnung zur Aerosoltherapie nach Anspruch 12, **dadurch gekennzeichnet, dass** das Smartphone einen Mikroprozessor umfasst, der eine spezielle Software ausführt, die Ressourcen des Smartphones, einschließlich der eingebetteten Kamera (21) und des Anzeigebildschirms (22) zum Erkennen und/oder Lesen des speziellen Musters (11) nutzt und in Reaktion ein zusammengesetztes Bild (23) auf dem Anzeigebildschirm (22) anzeigt, das ein Live-Bild des Gesichts eines Benutzers beim Inhalieren des Aerosols mittels des Medikationsinhalators (1) umfasst, das durch die spezielle Software "modifiziert" wird, um dem auf dem Anzeigebildschirm (22) erscheinenden Bild einen oder mehrere dynamische Inhalte hinzuzufügen, wie beispielsweise Tiere, Objekte, Figuren oder Umgebungselemente.

14. Anordnung zur Aerosoltherapie nach Anspruch 1, **dadurch gekennzeichnet, dass** sie ferner ein Gerät zur Aerosolmedikamentenabgabe (ADD) umfasst, das in den Medikationsinhalator (1) gesteckt ist.

## Revendications

1. Ensemble (1, 20) d'aérosolthérapie comportant un inhalateur (1) de médicament comportant un boîtier (2) définissant une chambre creuse (3), ledit boîtier (2) comportant en outre un couvercle inférieur (4) et un couvercle supérieur (5), ledit couvercle inférieur (4) comportant une entrée (6) destinée à y enficher un dispositif d'administration de médicaments en aérosol (ADD), ledit couvercle supérieur (5) comportant une sortie (7) en communication fluidique avec la chambre creuse (3), ledit couvercle inférieur (4) comportant en outre une surface inférieure externe (10), un motif spécifique (11) étant disposé sur ladite surface inférieure externe (10), ledit motif spécifique (11) pouvant être lu ou reconnu par un dispositif électronique (20),
**caractérisé en ce qu'**il comporte en outre un dispositif électronique (20) comportant une caméra incorporée (21) et un écran (22) d'affichage configuré pour :
a) reconnaître ou lire le motif spécifique porté par l'inhalateur (1) de médicament au moyen de la caméra incorporée (21),
b) exécuter un logiciel de modification d'image, comme une application, en réponse à une reconnaissance ou une lecture de l'étape a), et
c) afficher en direct sur l'écran d'affichage au moyen du logiciel de modification d'image, une image composite comportant une image en direct du visage de l'utilisateur et au moins un contenu dynamique superposé à et/ou autour de l'image en direct du visage de l'utilisateur.

2. Ensemble d'aérosolthérapie selon la revendication précédente, **caractérisé en ce que** le boîtier (2) de l'inhalateur (1) de médicament comporte une extrémité inférieure (2a) et une extrémité supérieure (2b), le couvercle inférieur (4) étant fixé de manière détachable à l'extrémité inférieure (2a) et/ou le couvercle supérieur (5) étant fixé de manière détachable à l'extrémité supérieure (2b).

3. Ensemble d'aérosolthérapie selon l'une quelconque des revendications précédentes, **caractérisé en ce que** le motif spécifique (11) est lisible par un dispositif électronique (20) comportant une caméra incorporée (21) et un écran (22) d'affichage.

4. Ensemble d'aérosolthérapie selon l'une quelconque des revendications précédentes, **caractérisé en ce que** le motif spécifique (11) est lisible par un ordiphone ou une tablette électronique.

5. Ensemble d'aérosolthérapie selon l'une quelconque des revendications précédentes, **caractérisé en ce que** le motif spécifique (11) comporte des formes géométriques ou non géométriques.

6. Ensemble d'aérosolthérapie selon l'une quelconque des revendications précédentes, **caractérisé en ce que** le motif spécifique (11) comporte plusieurs formes présentant des couleurs ou des contrastes différents pour être facilement reconnu ou lu par le dispositif électronique (20).

7. Ensemble d'aérosolthérapie selon l'une quelconque des revendications précédentes, **caractérisé en ce que** le motif spécifique (11) comporte des formes géométriques ou non géométriques présentant une orientation précise, permettant ainsi au dispositif électronique (20) de reconnaître l'orientation correcte de l'inhalateur (1) de médicament dans l'espace.

8. Ensemble d'aérosolthérapie selon l'une quelconque des revendications précédentes, **caractérisé en ce qu'**un clapet anti-retour (8) de l'inhalateur (1) de médicament est disposé entre le boîtier (2) et le couvercle supérieur (5).

9. Ensemble d'aérosolthérapie selon la revendication 8, **caractérisé en ce que** le clapet anti-retour (8) de l'inhalateur (1) de médicament est un clapet en bec de canard.

10. Ensemble d'aérosolthérapie selon l'une quelconque des revendications précédentes, **caractérisé en ce que** l'inhalateur (1) de médicament comporte en outre un élément (9) de sifflet.

11. Ensemble d'aérosolthérapie selon la revendication 10, **caractérisé en ce que** l'élément (9) de sifflet est disposé dans l'écoulement de gaz, entre le couvercle supérieur (5) et le boîtier (2) de l'inhalateur (1) de médicament.

12. Ensemble d'aérosolthérapie selon la revendication 1, **caractérisé en ce que** le dispositif électronique (20) est un ordiphone.

13. Ensemble d'aérosolthérapie selon la revendication 12, **caractérisé en ce que** l'ordiphone comporte un microprocesseur exécutant un logiciel spécifique qui utilise des ressources de l'ordiphone, y compris la caméra incorporée (21) et l'écran (22) d'affichage, pour reconnaître et/ou lire le motif spécifique (11) et affiche, en réponse, une image composite (23) sur l'écran (22) d'affichage, comportant une image en direct du visage de l'utilisateur, pendant l'inhalation de l'aérosol au moyen de l'inhalateur (1) de médicament, qui est "modifiée" par le logiciel spécifique pour ajouter un ou plusieurs contenus dynamiques, tels que des animaux, des objets, des figures ou des éléments environnementaux, sur l'image apparaissant sur l'écran (22) d'affichage.

14. Ensemble d'aérosolthérapie selon la revendication 1, **caractérisé en ce qu'**il comporte en outre un dispositif d'administration de médicaments en aérosol (ADD) enfiché dans l'inhalateur (1) de médicament.
